# EUROPEAN PATENT APPLICATION

(11) **EP 4 667 958 A1**
(43) Date of publication of application: **24.12.2025**
(21) Application number: 23924277.9
(22) Date of filing: 21.02.2023
(51) Int. Cl.: G01R 33/12, A61B 5/05

(54) **MAGNETIC SENSING DEVICE BASED ON DIAMOND NITROGEN-VACANCY SENSOR**

(71) Applicant: LG Electronics Inc., Yeongdeungpo-gu Seoul 07336 (KR)
(72) Inventor: LEE, Keunyoung, Seoul 06772 (KR); KIM, Seonghyok, Seoul 06772 (KR); LEE, Sungdan, Seoul 06772 (KR); KIM, Hongyeol, Seoul 06772 (KR); CHOI, Wookyoung, Seoul 06772 (KR); LEE, Donghun, Seoul 06772 (KR); PARK, Chanhu, Seoul 06772 (KR)
(74) Representative: Schott, Jakob Valentin
(86) International application number: PCT/KR2023/002464
(87) International publication number: WO 2024/177168

(57) **Abstract**

The present invention relates to a magnetic sensing device based on a diamond nitrogen-vacancy sensor. A magnetic sensing device based on a diamond nitrogen-vacancy sensor, according to one embodiment of the present invention, comprises: a non-quantum sensor for sensing a magnetic field signal; a quantum sensor that senses a magnetic field signal through the quantum phase of the electron spin of a negatively charged diamond nitrogen-vacancy center sensor within a diamond crystal; and a control unit that senses a magnetic field signal by means of the non-quantum sensor and the quantum sensor.

## Description

### Technical Field

The present disclosure relates to a magnetic sensing device based on a diamond nitrogen-vacancy sensor, and more particularly, to a device for sensing a magnetic field using a non-quantum sensor that senses a magnetic field and a quantum sensor including a diamond nitrogen-vacancy sensor.

### Background Art

Diamond crystals are made up of carbon atoms, but when carbon atoms are replaced by other types of atoms, lattice defects occur. One of the defects is a nitrogen-vacancy center, in which one carbon atom is replaced by a nitrogen atom and the neighboring carbon atom is removed, leaving a blank space.

A diamond nitrogen-vacancy center (DNV) can have an electron spin with spin number S of 1 so as to allow a spin quantum to have three spin states (ms) which are 1, 0, and -1. In the absence of an external magnetic field in an axial direction of the nitrogen-vacancy center in diamond, the spin quantum of spin states (ms) of +1 and - 1 overlap and exist at similar energy levels, but in the presence of an external magnetic field along the axial direction of the nitrogen-vacancy center in diamond, due to the Zeeman effect, the overlap of the spin quantum of the nitrogen-vacancy center in spin states (ms) of +1 and -1 disappears and the spin states exist at different energy levels, so the spin quantum of the nitrogen-vacancy center can have two resonance frequencies corresponding to spin transitions between the spin state (ms = 0) and the spin state (ms = +1) or between the spin state (ms = 0) and the spin state (ms = -1). A difference between the two resonance frequencies is proportional to a magnitude of the external magnetic field.

When a laser of 532 nm wavelength is irradiated on the diamond nitrogen-vacancy center, the quantum in the spin state (ms = 0) is excited and then returns to the ground state while emitting red light above 600 nm, and the quantum in the spin state (ms = +1) and spin state (ms = -1) is excited and then returns to the ground state while changing to the spin state (ms = 0) without emitting red light. Therefore, an amount of fluorescence of the emitted red light can be proportional to an amount of spin quantum in the spin state (ms = 0).

When two resonance frequencies corresponding to spin transitions are applied to the diamond nitrogen-vacancy center, a spin transition from the spin state (ms = 0) to the spin state (ms = +1) or the spin state (ms = -1) is caused, and accordingly, a quantum amount of the spin state (ms = 0) is reduced to reduce an amount of fluorescence of the emitted red light.

Therefore, when recording a change in amount of fluorescence according to frequency while applying a changing microwave frequency to the diamond nitrogen-vacancy center, an optically detected magnetic resonance (ODMR) spectrum with a reduced amount of fluorescence at the resonance frequency corresponding to each spin transition can be acquired. In the ODMR spectrum, a magnitude of the magnetic field applied to the diamond nitrogen-vacancy center can be determined based on a difference between the two resonance frequencies with a reduced amount of fluorescence.

Meanwhile, in order to sense a magnetic field signal coming from a body, a resolution at the femtotesla (10 to 15 T) level is required, and the only available sensor is a superconducting quantum interference device (SQUID) sensor for MRI, which is currently used to diagnose brain and heart diseases.

Since SQUID uses a superconductivity phenomenon, large additional systems such as a cryogenic system using liquid helium and liquid nitrogen to maintain a cryogenic environment are required.

Since a quantum sensor operates at room temperature with SQUID-level sensitivity, it is advantageous for small devices, and can be applied to low-maintenance MRI alternate brain and heart disease diagnosis apparatuses, and a technology that measures and analyzes a person's thought or state through brain signal detection/analysis and transmits it to a computer to operate objects according to intention, that is, a brain-computer interface (BCI).

For a BCI application, measurement and analysis must be performed immediately in everyday environments, rather than in strict measurements in spaces with limited external magnetic field signals, such as medical MRI.

### Disclosure of Invention

### Technical Problem

One aspect of the present disclosure is to provide a quantum sensor including a diamond nitrogen-vacancy sensor to which a lock-in amplifier technology is applied to a signal to reduce environmental noise.

Another aspect of the present disclosure is to provide a magnetic sensing device based on a diamond nitrogen-vacancy sensor that can improve magnetic field resolution using a non-quantum sensor that has been used to sense a magnetic field and a quantum sensor including a diamond nitrogen-vacancy sensor.

### Solution to Problem

In order to achieve the foregoing objectives, a magnetic sensing device based on a diamond nitrogen-vacancy sensor may include a non-quantum sensor for sensing a magnetic field signal, a quantum sensor that senses a magnetic field signal through a quantum phase of an electron spin of a negatively charged diamond nitrogen-vacancy sensor within a diamond crystal, and a controller that senses a magnetic field signal using the non-quantum sensor and the quantum sensor.

In one embodiment, the quantum sensor may include a diamond nitrogen-vacancy sensor, a frequency generator that outputs a reference frequency, a microwave generator that generates a microwave that causes a spin transition in the diamond nitrogen-vacancy sensor by modulating a frequency based on a reference frequency, a laser irradiator that applies a laser to excite a spin quantum to the diamond nitrogen-vacancy sensor, a detector that detects a fluorescence signal output from the diamond nitrogen-vacancy sensor, and a lock-in amplifier that determines a reference frequency to be output from the frequency generator based on a signal output from the detector.

In one embodiment, the quantum sensor may further include a first filter that transmits only a laser in a first wavelength range from the laser output from the laser irradiator, and a second filter that transmits only a fluorescence signal in a second wavelength range of the fluorescence signal output from the diamond nitrogen-vacancy sensor.

In one embodiment, the lock-in amplifier may extract an original signal component using trigonometric function characteristics.

In one embodiment, the quantum sensor may have a varying resolution according to a reference frequency in the same magnetic field region.

In one embodiment, the quantum sensor may have a better resolution as a range of change in a signal measured in the magnetic field region increases.

In one embodiment, the controller may divide a magnetic field region to be measured into a plurality of regions to determine a range of change in a magnetic field signal by the non-quantum sensor, and determine a reference frequency used when measuring by the quantum sensor based on the range of change in the signal determined in each of the plurality of regions.

In one embodiment, the controller may control the quantum sensor to perform magnetic sensing using a first reference frequency in a first region among the plurality of regions, and control the quantum sensor to perform magnetic sensing using a second reference frequency different from the first reference frequency in a second region from the first region among the plurality of regions.

In one embodiment, the controller may measure a magnetic field strength using the non-quantum sensor and the quantum sensor, and extract a signal to be measured using a signal measured by the non-quantum sensor from a signal including noise measured by the quantum sensor.

In one embodiment, the controller may measure a magnetic field strength using each of the non-quantum sensor and the quantum sensor regardless of the order of the non-quantum sensor and the quantum sensor.

### Advantageous Effects of Invention

According to the present disclosure, the present disclosure can provide a new magnetic field sensing method that can set a quantum sensor to an optimized measurement frequency for each magnetic field region to be measured using a non-quantum sensor to provide optimized resolution.

### Brief Description of Drawings

FIG. 1 is a view showing an energy level diagram of a diamond nitrogen-vacancy center.
FIG. 2 is a view showing an example of an optically detected magnetic resonance (ODMR) spectrum.
FIG. 3 is a view showing an example of applying a microwave frequency-modulated by a specific reference signal, and comparing phases of only reference signal components among signals generated from a photodetector to obtain an ODMR spectrum.
FIG. 4 is a view showing an example of a lock-in amplifier output.
FIG. 5 is a conceptual view for explaining a magnetic sensing device according to one embodiment of the present disclosure.
FIG. 6 is a conceptual view for explaining a quantum sensor including a diamond nitrogen-vacancy sensor according to one embodiment of the present disclosure.
FIGS. 7, 8, 9, 10, 11, and 12 are conceptual views for explaining that a magnetic field resolution varies depending on a measurement frequency of a diamond nitrogen-vacancy sensor according to one embodiment of the present disclosure.
FIG. 13 is a flowchart for explaining a method for sensing a magnetic field using a non-quantum sensor and a quantum sensor according to one embodiment of the present disclosure.
FIG. 14 is a conceptual view showing a method for sensing a magnetic field using a non-quantum sensor and a quantum sensor according to another embodiment of the present disclosure.

### Mode for the Invention

Hereinafter, embodiments disclosed in this specification will be described in detail with reference to the accompanying drawings, and the same or similar elements are designated with the same reference numerals regardless of the drawing numerals and redundant description thereof will be omitted. The suffixes "module" and "portion" for elements used in the following description are given or used interchangeably in consideration of only the ease of writing the specification, and do not have meanings or roles that are distinguished from each other by themselves. In addition, in describing embodiments disclosed in this specification, when it is determined that a detailed description of related known technologies can obscure the subject matter of the embodiments disclosed in this specification, the detailed description thereof will be omitted. In addition, the accompanying drawings are provided only for a better understanding of the embodiments disclosed in this specification and are not intended to limit the technical concept disclosed in this specification, and therefore, it should be understood that the accompanying drawings include all modifications, equivalents and substitutes within the concept and technical scope of the present disclosure.

The terms including an ordinal number such as first, second, and the like can be used to describe various elements, but the elements should not be limited by those terms. The terms are used merely for the purpose to distinguish one element from another element.

It should be understood that when an element is referred to as being "connected to" or "coupled to" another element, the element can be directly connected to or coupled to the other element or intervening elements can also be present. On the contrary, it should be understood that when an element is referred to being "directly connected" or "directly coupled" to another element, there are no intervening elements present.

A singular representation can include a plural representation, unless the context clearly indicates otherwise.

The terms "include" or "have" used herein should be understood that they are intended to indicate the presence of a feature, a number, a step, an operation, an element, a component or a combination thereof disclosed in the specification, and the presence or additional possibility of one or more other features, numbers, steps, operations, elements, components or combinations thereof are not excluded in advance.

A magnetic sensing device based on a diamond nitrogen-vacancy sensor described in this specification can be implemented in a form of a cellular phone, a smart phone, a laptop computer, a digital broadcast terminal, a personal digital assistant (PDA), a portable multimedia player (PMP), a navigator, a slate PC, a tablet PC, an ultrabook, a wearable device (e.g., a smartwatch, smart glasses, a head mounted display (HMD)), or the like.

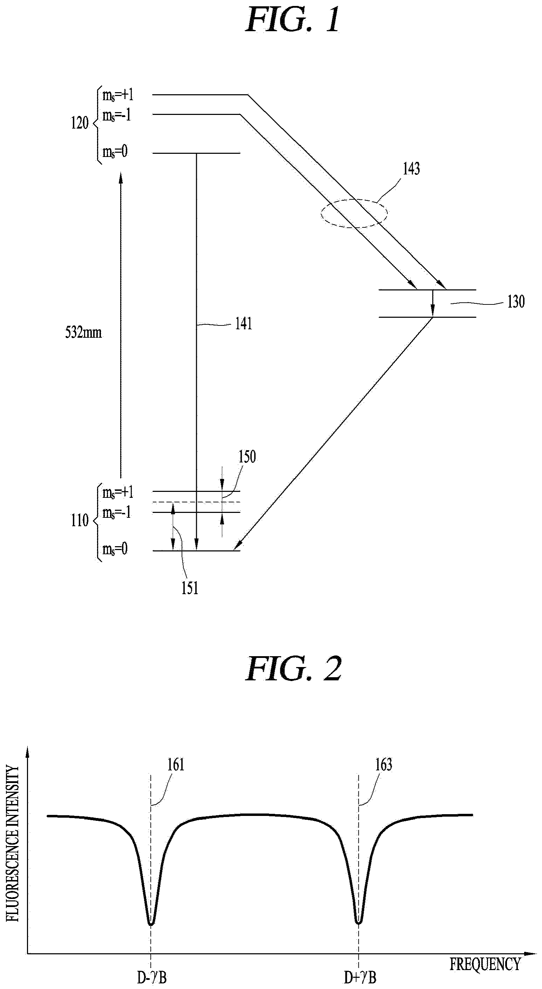

FIG. 1 is a view showing an energy level diagram of a diamond nitrogen-vacancy center.

Referring to FIG. 1, a spin quantum of a diamond nitrogen-vacancy center has a ground state 210 that is a spin triplet having three spin states: a spin state (ms = 0), and a spin state and a spin state (ms = -1) that are symmetrical to each other. In the absence of a magnetic field, the spin state (ms = +1) and the spin state (ms = -1) are in the same energy state, which is a predetermined energy level away from the spin state (ms = 0), due to a spin-spin interaction. In the absence of a magnetic field, an energy level of the spin state (ms = 0) is separated from energy levels of the spin state (ms = +1) and spin state (ms = -1) by an energy of approximately 2.87 GHz.

When an external magnetic field is applied to the ground state 210, the energy of the spin state (ms = +1) and the spin state (ms = -1) that has had the same energy is separated into energy levels 250 in proportion to a magnitude of the applied external magnetic field.

The spin quantum of the diamond nitrogen-vacancy center in the ground state 210 is excited to an excited state 220 when irradiated with green light. The green light can be light having a wavelength below 637 nm, but preferably light having a wavelength of 532 nm. At this time, the spin quantum of the diamond nitrogen-vacancy center is excited while maintaining its spin state.

Here, the spin quantum in the excited state 220 returns to the ground state 210, and the spin quantum in the spin state (ms = 0) mostly returns to the ground state 210 of the spin state (ms = 0) while emitting photons of red light (e.g., above 600 nm and below 900 nm) (241), and partly returns to the ground state 210 by passing through a singlet 230 (243), and returns to the ground state 210 without emitting red light when passing through the singlet 230.

The spin quantum of the diamond nitrogen-vacancy center in the spin state (ms = +1) and spin state (ms = -1) in the excited state 220 mostly returns to the ground state 210 through the singlet 230, and returns, when returning, to the spin state (ms = 0) rather than the original spin state (ms = ±1). Therefore, when irradiated with green light, subsequent to a predetermined period of time, the spin quantum of the diamond nitrogen-vacancy center mostly has the spin state (ms = 0).

Meanwhile, when a resonance frequency corresponding to an energy difference between the spin state (ms = 0) and the spin state (ms = +1) or between the spin state (ms = 0) and the spin state (ms = -1) is applied, a spin transition from the spin state (ms = 0) of the ground state 210 to the spin state (ms = +1) or the spin state (ms = -1) is induced. Under an external magnetic field (B), each resonance frequency (F) is determined by F = D ± ηB. Here, D is a resonance frequency in the absence of a magnetic field, that is, a zero-field separation resonance frequency, which can have a value of 2.87 GHz, and η is an electron spin gyromagnetic ratio, which can have a value of 28 MHz/mT.

When green light (e.g., light with a wavelength of 532 nm) is applied to the spin quantum of the ground state 210 of the diamond nitrogen-vacancy center, it is excited to the excited state 220, and the spin quantum of the spin state (ms = 1) and the spin state (ms = -1) of the excited state 220 mostly returns to the ground state 210 without emitting red light through 243 of FIG. 1, and the spin quantum of the spin state (ms = 0) of the excited state 220 mostly returns to the ground state 210 while emitting red light above 600 nm along 241 of FIG. 1. At this time, the spin quantum of the spin state (ms = ±1) returns to the ground state 210 while being converted to the spin state (ms = 0), and the spin quantum of the spin state (ms = 0) returns to the ground state 210 while maintaining the spin state.

Therefore, when an optically detected magnetic resonance (ODMR) spectrum is recorded by changing a wavelength of the applied microwave and recording a change in an amount of emitted light using a photodiode, a portion with a low amount of light can be observed at two resonance frequencies corresponding to an energy difference between the spin state (ms = 0) and the spin state (ms = +1) and an energy difference between the spin state (ms = 0) and the spin state (ms = -1).

FIG. 2 is a view showing an example of an ODMR spectrum.

Referring to FIG. 2, the ODMR spectrum shows a low amount of light measured at two resonance frequencies corresponding to an energy difference 263 between the spin state (ms = 0) and the spin state (ms = +1) or an energy difference 261 between the spin state (ms = 0) and the spin state (ms = -1). When a frequency other than the corresponding resonance frequency is applied, the spin state (ms = 0) of the ground state 210 is not excited to the spin state (ms = +1) or the spin state (ms = -1) and remains in the spin state (ms = 0). Furthermore, when excited to the excited state 220 by green light, the spin quantum of the diamond nitrogen-vacancy center, which has remained in the spin state (ms = 0), returns to the ground state 210 by emitting photons of red light along the path 241 of FIG. 1. Conversely, when the corresponding resonance frequency is applied, the spin quantum of the spin state (ms = 0) in the ground state 210 is converted to the spin state (ms = +1) or the spin state (ms = -1), and then excited again by green light to the excited state 220, and then returns to the ground state 210 without emitting photons of red light through the path 243 of FIG. 1, and therefore, an amount of light measured by the photodiode decreases. Therefore, when measuring the ODMR spectrum, an amount of light measured at two resonance frequencies corresponding to the energy difference 263 between the spin state (ms = 0) and the spin state (ms = +1) and the energy difference 261 between the spin state (ms = 0) and the spin state (ms = -1) is low. Furthermore, a difference between those two frequency bands is proportional to a strength of a magnetic field applied to the diamond nitrogen-vacancy center. Therefore, the diamond nitrogen-vacancy center can be used to detect an existing magnetic field or a change in the magnetic field.

When obtaining an ODMR spectrum such as that shown in FIG. 2, a frequency modulation technique can be used to improve a signal-to-noise ratio.

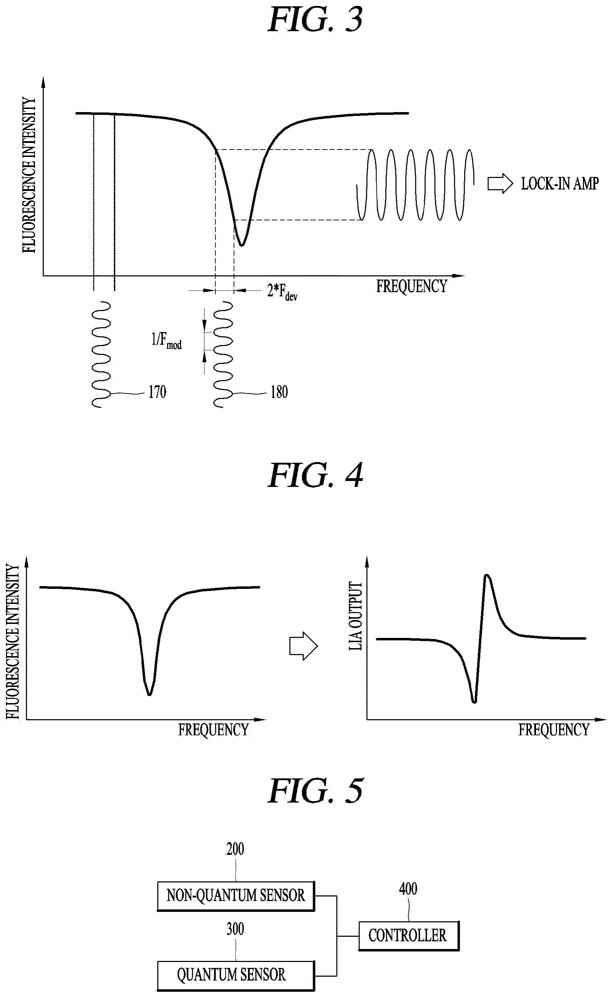

FIG. 3 is a view showing an example of applying a microwave frequency-modulated by a specific reference signal, and comparing phases of only reference signal components among signals generated from a photodetector to obtain an ODMR spectrum, and FIG. 4 is a view showing an example of a lock-in amplifier output.

Referring to FIG. 3, when a microwave 170 that has been frequency-modulated with a reference signal at a frequency other than a resonance frequency is applied, the slope is almost zero, and therefore, a signal generated from the photodetector has almost no reference signal component. On the contrary, when a microwave 180 that has been frequency-modulated with a reference signal at a resonance frequency is applied, the slope in the relevant region becomes large, and therefore, a reference signal component appears in a signal detected by the photodetector.

Therefore, a reference signal can be detected by using a lock-in amplifier (LIA) that can extract a frequency of the reference signal. Using frequency modulation as shown in FIG. 3, the reference signal can be relatively raised, thereby improving a signal-to-noise ratio by avoiding the influence of noise existing in a low frequency band of the signal.

As can be seen from FIG. 3, a magnitude of a signal input to a lock-in amplifier signal is proportional to a slope of an ODMR spectrum, so a lock-in amplifier signal output obtained through a frequency modulation method has a differential form of an original ODMR spectrum and becomes a signal output as shown in FIG. 4. Therefore, when detecting a change in the lock-in amplifier output as shown in FIG. 4, a change in an external magnetic field can be detected.

Meanwhile, the present disclosure can provide a magnetic sensing device capable of significantly improving a resolution of sensing a magnetic field by using not only a quantum sensor including a diamond nitrogen-vacancy sensor but also a non-quantum sensor (e.g., a magnetic sensor) that has been used to sense a magnetic field.

FIG. 5 is a conceptual view for explaining a magnetic sensing device according to one embodiment of the present disclosure.

The magnetic sensing device of the present disclosure can include a non-quantum sensor 200 for sensing a magnetic field signal, a quantum sensor 300 that senses a magnetic field signal through a quantum phase of an electron spin of a negatively charged diamond nitrogen-vacancy sensor within a diamond crystal, and a controller 400 that senses a magnetic field signal using the non-quantum sensor and the quantum sensor.

The controller 400 can implement a magnetic sensing technology capable of wide bandwidth and precise measurement through a hybrid sensing method that uses the non-quantum sensor 200 and the quantum sensor 300 together.

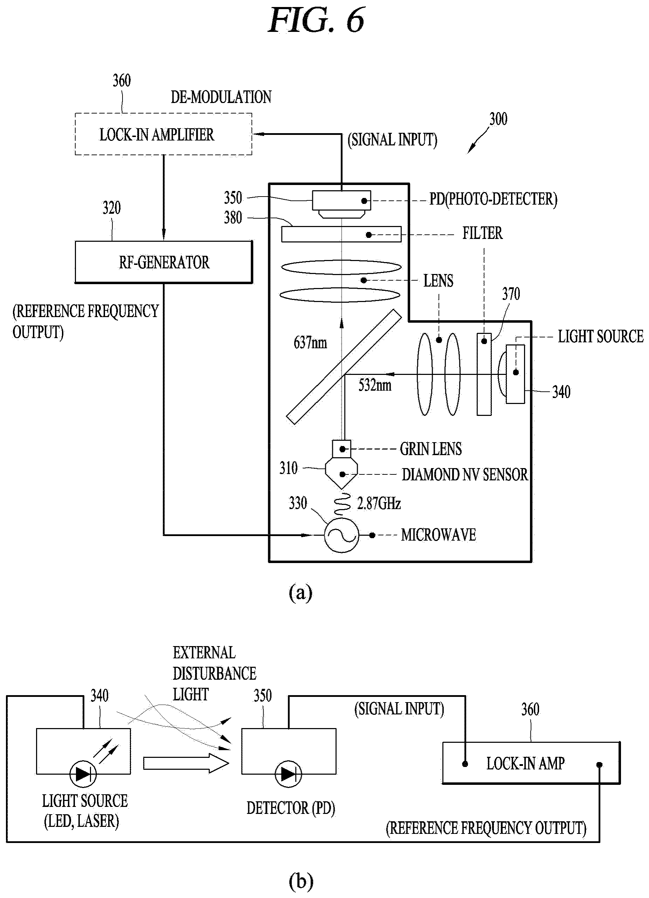

FIG. 6 is a conceptual view for explaining a quantum sensor including a diamond nitrogen-vacancy sensor according to one embodiment of the present disclosure.

Referring to (a) of FIG. 6, the quantum sensor 300 included in the magnetic sensing device of the present disclosure can include a diamond nitrogen-vacancy sensor 310, a frequency generator (radio frequency (RF)-generator) 320 that outputs a reference frequency (or reference signal), a microwave generator 330 that modulates a frequency based on the reference frequency to generate a microwave that causes a spin transition in the diamond nitrogen-vacancy sensor, a laser irradiator 340 that applies a laser to excite a spin quantum to the diamond nitrogen-vacancy sensor, a detector (photo-detector (PD)) 350 that detects a fluorescence signal output from the diamond nitrogen-vacancy sensor, and a lock-in amplifier 360 that determines a reference frequency to be output from the frequency generator based on a signal output from the detector.

In addition, the quantum sensor 300 can further include a first filter 370 that transmits only a laser in a first wavelength range (e.g., a laser of a wavelength of 532 nm) output from the laser irradiator, and a second filter 380 that transmits only a fluorescence signal in a second wavelength range (e.g., a fluorescence signal that outputs red light above 600 nm) output from the diamond nitrogen-vacancy sensor.

The frequency generator (or reference frequency generator) 320 can generate a reference signal to be used for frequency modulation. According to one embodiment, the reference frequency can be a frequency between 1 KHz and 100 KHz. The frequency generator 320 can transmit a reference signal generated by the microwave generator 330 and the lock-in amplifier 360. A reference signal can be referred to as a signal having a reference frequency. The reference signal transmitted to the microwave generator 330 can also be transmitted to the lock-in amplifier 360 to be compared with a signal to be detected by the lock-in amplifier 360.

The microwave generator 330 can generate a microwave obtained by modulating a microwave having a first resonance frequency that causes a spin transition of a spin quantum of the diamond nitrogen-vacancy sensor 310 from a spin state (ms = 0) to a spin state (ms = -1) and a microwave having a second resonance frequency that causes a spin transition from a spin state (ms = 0) to a spin state (ms = +1) based on a reference signal.

The quantum sensor can further include a power amplifier (not shown) that amplifies a microwave signal generated by the microwave generator 330 to input it to the diamond nitrogen-vacancy sensor 310.

The laser irradiator 340 can irradiate a laser that excites a spin quantum from a ground state 210 to an excited state 220 to the diamond nitrogen-vacancy sensor 310. The wavelength of the laser can be, for example, 532 nm.

The spin quantum of the diamond nitrogen-vacancy sensor 310 can cause two spin transitions by a microwave signal generated and input by the microwave generator 330, and can be excited to the excited state 220 by a laser irradiated by the laser irradiator 340 and then returned to the ground state 210 to produce fluorescence.

The lock-in amplifier 360 can receive a fluorescence signal generated from the diamond nitrogen-vacancy sensor 310 to output a result of comparison with a reference signal.

Referring to (b) of FIG. 6, an environment in which a magnetic field is measured is such that there exists a signal S to be measured and a noise signal N, and a signal measured by the detector is a signal that is a sum of the signal S and the signal N (S + N).

The lock-in amplifier 360 can extract an original signal component from a fluorescence signal (S + N) detected by the detector 350 by using trigonometric function characteristics.

That is, the lock-in amplifier 360 can extract the original signal component by using trigonometric function characteristics.

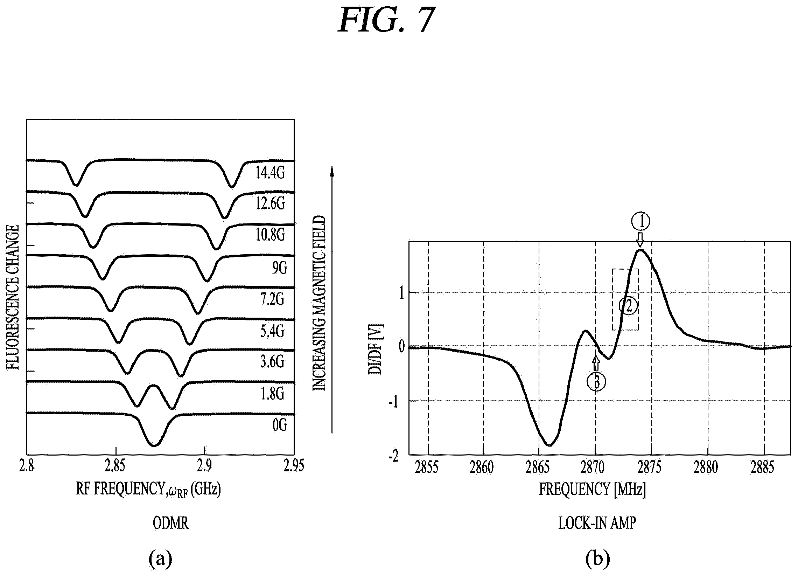

FIGS. 7, 8, 9, 10, 11, and 12 are conceptual views for explaining that a magnetic field resolution varies depending on a measurement frequency of a diamond nitrogen-vacancy sensor according to one embodiment of the present disclosure.

Referring to an ODMR spectrum shown in (a) of FIG. 7, it can be confirmed that a resonance frequency varies as a strength of a magnetic field increases.

As described above, a magnitude of a signal input to a lock-in amplifier signal is proportional to a slope of an ODMR spectrum, so a lock-in amplifier signal output obtained through a frequency modulation method has a differential form of an original ODMR spectrum and becomes a signal output as shown in (b) of FIG. 7.

Therefore, when detecting a change in the lock-in amplifier output as shown in (b) of FIG. 7, a change in an external magnetic field can be detected.

Meanwhile, in the present disclosure, a lock-in amplifier technology can be used to detect a magnetic field below a predetermined strength that is difficult to detect/distinguish with ODMR. In this case, the lock-in amplifier technology causes a difference in sensing resolution according to a strength of a magnetic field and a reference frequency determined by a lock-in amplifier.

Specifically, the quantum sensor can have a varying resolution according to a reference frequency in the same magnetic field region.

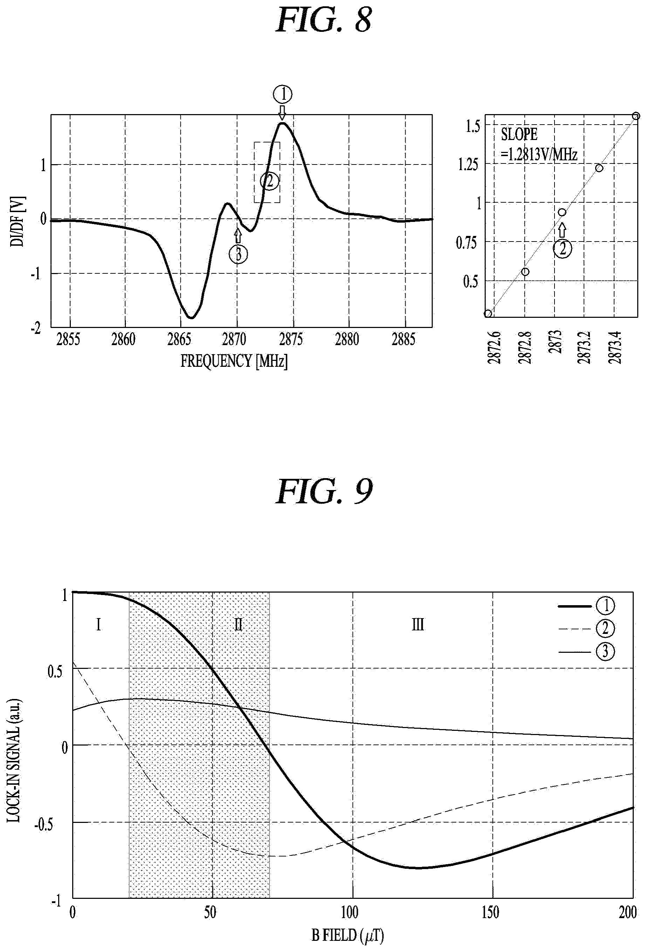

For example, as shown in FIG. 8, when a reference frequency such as ①, ②, and ③ is set differently in the lock-in amplifier 360, an output result of the lock-in amplifier varies as shown in FIG. 9.

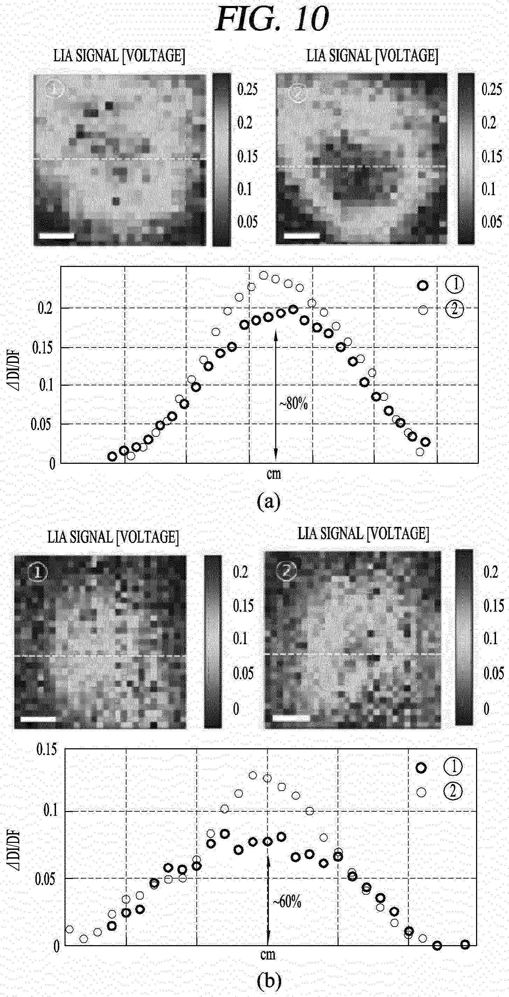

Referring to (a) of FIG. 10, it can be confirmed that more red light is detected in the output result of the lock-in amplifier on the right when the reference frequency of ② is used than in the output result of the lock-in amplifier on the left when the reference frequency of ① is used, and it can also be confirmed in (b) of FIG. 10 that there is a difference in resolution according to a varying reference frequency.

The quantum sensor 300 can have a better resolution as a range of change in a signal measured in the magnetic field region increases.

Specifically, the controller 400 can divide a magnetic field region to be measured into a plurality of regions to determine a range of change in a magnetic field signal by the non-quantum sensor, and determine a reference frequency used when measuring by the quantum sensor based on the range of change in the signal determined in each of the plurality of regions.

For example, the controller 400 can control the quantum sensor to perform magnetic sensing using a first reference frequency (a reference frequency of ② having a largest range of change in a region 1100) in the first region (I) 1100 among the plurality of regions (I, II, III).

In addition, the controller 400 can control the quantum sensor to perform magnetic sensing using a second reference frequency (a reference frequency of ① having a largest range of change in a region 1200) different from the first reference frequency in a second region (an intermediate region between II and III) 1200 different from the first region among the plurality of regions (I, II, III).

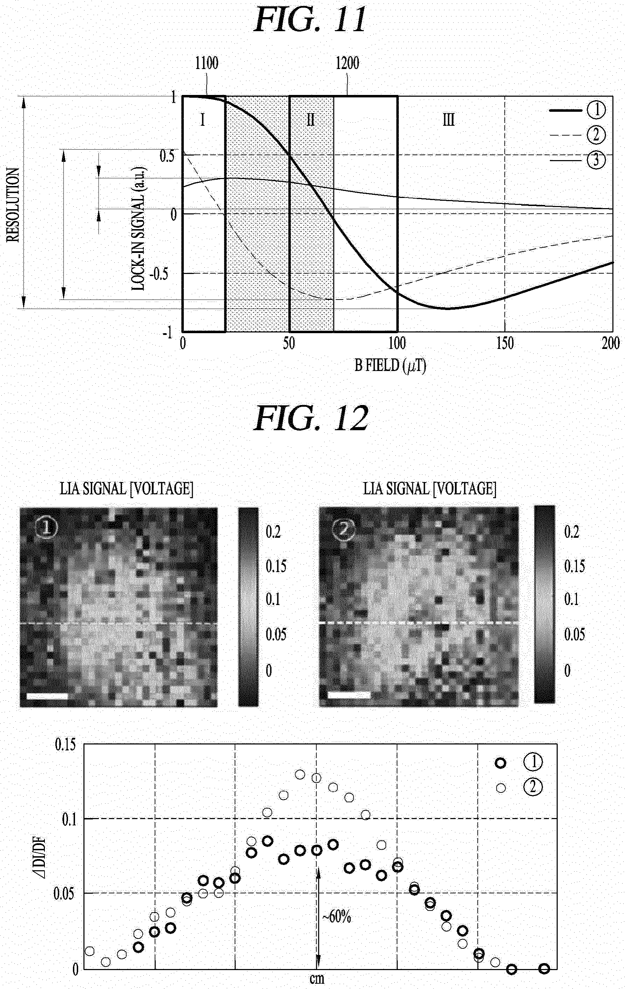

Referring to FIG. 12, it can be confirmed that in a result in the first region, more red is detected when the reference frequency of ② is used than when the reference frequency of ① is used, and thus the resolution is improved.

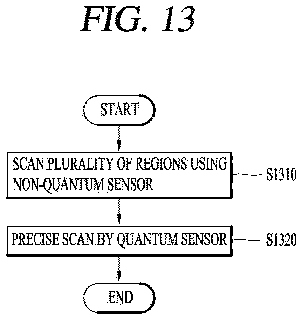

FIG. 13 is a flowchart for explaining a method for sensing a magnetic field using a non-quantum sensor and a quantum sensor according to one embodiment of the present disclosure.

As described above, the present disclosure can scan a magnetic field for each of a plurality of regions using a non-quantum sensor (existing magnetic sensor) (S1310), and then determine a reference frequency used in a quantum sensor to perform a precise scan (S1320), thereby significantly improving the resolution when sensing a magnetic field.

Meanwhile, the magnetic sensing device of the present disclosure can extract a signal to be measured using a sensing result subsequent to sensing simultaneously, rather than scanning with a non-quantum sensor and then scanning with a quantum sensor.

Specifically, the controller 400 can measure a magnetic field strength using the non-quantum sensor and the quantum sensor, and extract a signal to be measured using a signal measured by the non-quantum sensor from a signal including noise measured by the quantum sensor.

In this case, the controller 400 can measure a magnetic field strength using each of the non-quantum sensor and the quantum sensor, regardless of the order of the non-quantum sensor and the quantum sensor.

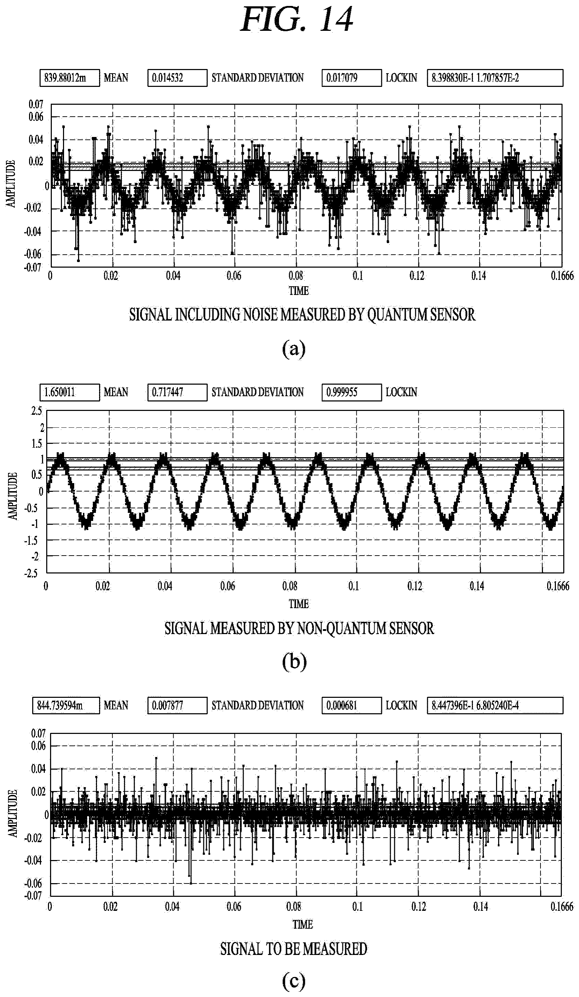

FIG. 14 is a conceptual view showing a method for sensing a magnetic field using a non-quantum sensor and a quantum sensor according to another embodiment of the present disclosure.

(a) of FIG. 14 can include a signal including noise measured by a quantum sensor, and (b) of FIG. 14 can include a signal measured by a non-quantum sensor.

The controller 400 can simultaneously measure a magnetic field strength using a non-quantum sensor and a quantum sensor, thereby excluding a large noise signal with a magnitude of µT to nT measured by the non-quantum sensor from a signal including various noises. Through this, the present disclosure can improve magnetic sensing performance.

While the preferred embodiments of the present disclosure have been shown and described above, it will of course be understood by those skilled in the art that various modifications can be made without departing from the subject matter of the disclosure as defined in the following claims, and it is to be noted that those modifications should not be understood individually from the technical concept and prospect of the present disclosure.

## Claims

1. A magnetic sensing device based on a diamond nitrogen-vacancy sensor, the device comprising:
a non-quantum sensor for sensing a magnetic field signal;
a quantum sensor that senses a magnetic field signal through a quantum phase of an electron spin of a negatively charged diamond nitrogen-vacancy sensor within a diamond crystal; and
a controller that senses a magnetic field signal using the non-quantum sensor and the quantum sensor.

2. The device of claim 1, wherein the quantum sensor comprises:
a diamond nitrogen-vacancy sensor;
a frequency generator that outputs a reference frequency;
a microwave generator that generates a microwave that causes a spin transition in the diamond nitrogen-vacancy sensor by modulating a frequency based on a reference frequency;
a laser irradiator that applies a laser to excite a spin quantum to the diamond nitrogen-vacancy sensor;
a detector that detects a fluorescence signal output from the diamond nitrogen-vacancy sensor; and
a lock-in amplifier that determines a reference frequency to be output from the frequency generator based on a signal output from the detector.

3. The device of claim 2, wherein the quantum sensor further comprises:
a first filter that transmits only a laser in a first wavelength range from the laser output from the laser irradiator; and
a second filter that transmits only a fluorescence signal in a second wavelength range of the fluorescence signal output from the diamond nitrogen-vacancy sensor.

4. The device of claim 1, wherein the lock-in amplifier extracts an original signal component using trigonometric function characteristics.

5. The device of claim 2, wherein the quantum sensor has a varying resolution according to a reference frequency in the same magnetic field region.

6. The device of claim 5, wherein the quantum sensor has a better resolution as a range of change in a signal measured in the magnetic field region increases.

7. The device of claim 1, wherein the controller divides a magnetic field region to be measured into a plurality of regions to determine a range of change in a magnetic field signal by the non-quantum sensor, and determines a reference frequency used when measuring by the quantum sensor based on the range of change in the signal determined in each of the plurality of regions.

8. The device of claim 7, wherein the controller controls the quantum sensor to perform magnetic sensing using a first reference frequency in a first region among the plurality of regions, and controls the quantum sensor to perform magnetic sensing using a second reference frequency different from the first reference frequency in a second region from the first region among the plurality of regions.

9. The device of claim 1, wherein the controller measures a magnetic field strength using the non-quantum sensor and the quantum sensor, and extracts a signal to be measured using a signal measured by the non-quantum sensor from a signal including noise measured by the quantum sensor.

10. The device of claim 9, wherein the controller measures a magnetic field strength using each of the non-quantum sensor and the quantum sensor regardless of the order of the non-quantum sensor and the quantum sensor.
